(19)

Europäisches
Patentamt
European
Patent Office
Office européen
des brevets

(11) **EP 3 306 500 A1**

(12) **EUROPEAN PATENT APPLICATION**
published in accordance with Art. 153(4) EPC

(43) Date of publication:
**11.04.2018 Bulletin 2018/15**

(21) Application number: **16802535.1**

(22) Date of filing: **31.05.2016**

(51) Int Cl.:
***G06F 19/00*** (2018.01)

(86) International application number:
**PCT/CN2016/084000**

(87) International publication number:
**WO 2016/192612 (08.12.2016 Gazette 2016/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **02.06.2015  CN 201510294286**

(71) Applicant: **Chen, Kuan
Beijing 100011 (CN)**

(72) Inventor: **Chen, Kuan
Beijing 100011 (CN)**

(74) Representative: **Prol European Patent Attorneys
Postfach 2123
90711 Fürth (DE)**

(54) **METHOD FOR ANALYSING MEDICAL TREATMENT DATA BASED ON DEEP LEARNING, AND INTELLIGENT ANALYSER THEREOF**

(57)  Disclosed is a method for analyzing medical data based on deep learning which is capable of effectively reducing work load of doctors or medical researchers in hospitals and is capable of scientifically analyzing masses of medical treatment or medical data and acquiring an analysis result matching the data, and an intelligent analyzer thereof. The core idea of the method and the intelligent analyzer according to the present invention is to establish a model in a computer by using the deep convolution neural network algorithm in the deep learning. This model selects and optimizes the model parameter by using the masses of medical data, and automatically learns the pathological analysis course of the doctors and medical researchers by means of a "training" model and hence helps them to process the masses of medical treatment or medical data. Under the assistance, finally the doctors make correct judgments and effective decisions with respect to the masses of medical data. The present invention may greatly reduce the work load of the doctors or medical researchers, and improve the working efficiency thereof. According to the present invention, the doctors or medical researchers may be relieved from the overloaded medical treatment or medical data analysis work, such that they contribute much to more important work.

FIG. 1

**Description**

**TECHNICAL FIELD**

The present invention relates to intelligent devices for analyzing medical treatment or medical data, and in particular, relates to an intelligent analyzer which automatically aggregates masses of medical treatment or medical data acquired by large-scale hospitals or medical research organizations, and gives analysis results matching the data.

**BACKGROUND**

Generally, doctors in hospitals or researchers in medical research organizations need to complete a lot of work each day. For example, doctors in clinical departments in hospitals need to analyze and research the acquired medical data, and make a decision. The following data is the medical data randomly extracted and generated in one day in a third grade A hospital in Beijing.

1162 CT reports, 1461 X-ray reports, 325 MRI reports, wherein each X-ray report includes 2 two-dimensional images, each CT report averagely includes 50 two-dimensional images, and each MRI report averagely includes 100 two-dimensional images. With respect to all the examination data, doctors in the radiology departments need to draft reports. However, a large-scale third grade A hospital generally has 20 radiological doctors among which there are old doctors that are not good at typewriting and young doctors that are lack in experience. Therefore, the great work load each day may exert a great pressure for the doctors and researchers, and thus their energy is quickly reduced. In this way, the working efficiency thereof is low, and even the analysis results have a high error rate.

**SUMMARY**

The technical problem to be solved by the present invention is to provide a method for analyzing medical data based on deep learning which is capable of effectively reducing work load of doctors or medical researchers in hospitals and is capable of scientifically analyzing masses of medical treatment or medical data and acquiring an analysis result matching the data, and an intelligent analyzer thereof.

To solve the above technical problem, the present invention employs the following technical solution:

A method for analyzing medical data based on deep learning according to the present invention includes the following steps:

1) acquiring masses of docketed medical material data of the same category and medical diagnosis data matching the medical material data as medical training data, and storing the medical training data in a computer;

2) associating image data that is not less than two dimensions in the medical training data with variation values variable with time and space in textual data and corresponding data;

3) in the acquired masses of docketed medical material data, aggregating the medical training data corresponding to each individual and the variation value as unit data;

4) integrating or formatting the medical training data into a computer-understandable structured data matrix by means of segmentation, association or textual data mining, and extracting a data feature from each unit data;

5) importing the medical training data that is formed to the structured data matrix into a memory module in a computer corresponding to a deep learning model;

6) performing an optimization operation for the deep learning model via the computer as follows:

a. setting a basic deep learning framework, and establishing a data model including an input layer, at least one hidden layer and an output layer for the medical training data according to the data feature, wherein the input layer includes several nodes having a data feature, the output layer includes several nodes having a medical diagnosis data feature, and each hidden layer includes several nodes having a mapping relationship with an upper-layer output value;

b. establishing a data model for each node by using a mathematic equation, and presetting a relevant parameter value Ai in the mathematic equation in a manual or random way, wherein an input value of each node in the input layer is the data feature, an input value of each node in the hidden layers and the output layer is an upper-layer output value, and an output value of each node in all the layers is a value of the node experiencing the mathematic equation operation; and

c. initializing the parameter value Ai, comparing the output value of each node in the output layer with the medical diagnosis data feature of the corresponding node, repeatedly rectifying the reference value Ai of each node, and performing the operation cyclically, such that the parameter value Ai of each node that causes a similarity between a similarity between the output value of each node in the output layer and the medical diagnosis data feature to be a locally maximum value is acquired;

7) importing to-be-analyzed medical data formed as the structured matrix data into the deep learning model for a medical pathological analysis matching the to-be-analyzed medical data; and

8) outputting a medical pathological analysis result matching the to-be-analyzed medical data by the deep learning model via an output apparatus.

The parameter value Ai is optimized by using a non-supervised leaning approach.

The non-supervised leaning approach employs a denoising autoencoder or a restricted Boltzmann machine for self-learning.

The parameter value Ai is optimized by using a supervised leaning approach.

The mathematical equation is a parameter mathematical equation or a non-parameter mathematical equation; wherein the parameter mathematical equation is a linear model, a neural network model or a convolution operation, the non-parameter mathematical equation is an extremum operation equation, and a mathematical model is defined as follows:

$$y=g(X)=fn \circ fn\text{-}1 \circ fn\text{-}2 \circ \ldots \circ f1(X)$$

wherein y denotes a medical diagnosis data feature in the output layer having a dimension of $M_n$, X denotes training material data having a dimension of $M_0$, and $f_1$ to fn denote defined operation equations in each layer, wherein equation $f_i$ of each layer has a dimension of $M_i\text{-}1\text{->}M_i$, equation $f_1$ of the first layer converts the X having the dimension of $M_0$ into an output $Z_1$ having a dimension of $M_1$, $Z_1$ is an input of equation $f_2$ of the second layer, and the like, wherein model $f_i$ of each layer model has a matched parameter group $A_i$.

The medical material data includes relevant information recorded in terms of diagnosis, examination and treatment for patients by doctors at clinical and medical technique stages; and the diagnosis data includes relevant information recorded in terms of preliminary diagnosis, hospital discharge result, disease treatment effect for the patients by the doctors at the clinical and medical technique stages, and textual visit data and tracking visit data documented by the doctors.

The data feature includes variation values variable of the medical training data in terms of time and space, and various mathematic statistical values of the medical training data, for example, a tendency of data rise or falloff with the time.

The structured data relevant to the to-be-analyzed medical data and the matched medical pathological analysis result is fed back to the deep learning model to form new training data.

An intelligent analyzer for analyzing medical data based on deep learning according to the present invention includes: an input apparatus configured to import medical training data and to-be-analyzed medical data into a computer, a memory module configured to respectively or integrally store the medical training data and the to-be-analyzed medical data, a deep learning model module configured to call the medical training data in the memory module for self-learning, an output apparatus configured to export a medical pathological analysis result matching the to-be-analyzed medical data, and a processor including a CPU and/or a GPU; wherein

the medical training data includes medical material data and medical pathological data matching the medical training data;

the medical training data and the to-be-analyzed medical data are a computer-understandable structured data matrix;

the self-learning employs a parameter mathematical equation including a linear model, a neural network model, a convolution operation and/or a maximum value operation;

the input apparatus includes a computer device deployed in a hospital or a medical organization, and various medical examination devices and pathological analysis devices that are connected to the computer device; and

the output apparatus includes a fixed computer output terminal and a mobile intelligent terminal that are deployed in the hospital or the medical organization and connected to the input apparatus.

The intelligent analyzer according to the present invention further includes a network connection module that is connectable to the Internet and Ethernet, wherein the network connection module supports optical fiber connection, WiFi connection or GPRS module connection.

The core idea of the method and the intelligent analyzer according to the present invention is to establish a model in a computer by using the deep convolution neural network (DCCN) algorithm in the deep learning. This model selects and optimizes the model parameter by using the masses of medical data, and automatically learns the pathological analysis course of the doctors and medical researchers by means of a "training" model and hence helps them to process the masses of medical treatment or medical data. Under the assistance, finally the doctors make correct judgments and effective decisions with respect to the masses of medical data. The present invention may greatly reduce the work load of the doctors or medical researchers, and improve the working efficiency thereof. According to the present invention, the doctors or medical researchers may be relieved from the overloaded medical treatment or medical data analysis work, such that they contribute much to more important work.

## BRIEF DESCRIPTION OF THE DRAWINGS

FIG. 1 is a block diagram illustrating working of an intelligent analyzer according to the present invention;
FIG. 2 illustrates image data generated by means of brain MRI;
FIG. 3 illustrates image data generated after a target object is deleted from the image data;
FIG. 4 is a schematic diagram illustrating graphic data-based DNN basic mathematical construction;
FIG. 5 is a schematic operation diagram of a convolution block;
FIG. 6 is a schematic logic operation diagram of an interconnected multi-layer interceptron; and
FIG. 7 is a schematic working flowchart of a denoising autoencoder.

## DETAILED DESCRIPTION

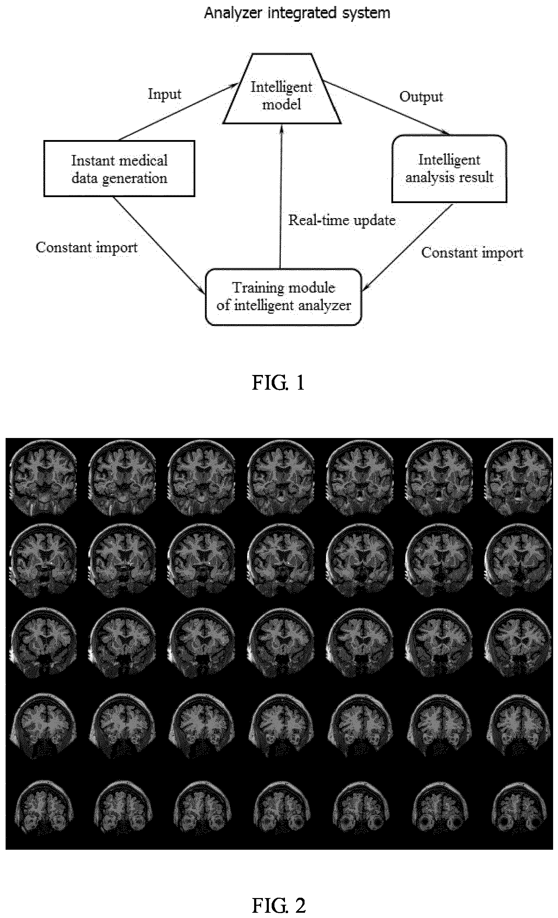

As illustrated in FIG. 1, the method for analyzing medical data based on deep learning according to the present invention selects and optimizes the model parameter by using the masses of medical data, and automatically learns the pathological analysis course of the doctors and medical researchers by means of a "training" model and hence helps them to process the masses of medical treatment or medical data. Under the assistance, finally the doctors make correct judgments and effective decisions with respect to the masses of medical data.

Generally, a medical data intelligent analysis system is very important in the field of medical science. For example, in the aspect of medical imaging data, many researchers are studying the subject of CT pulmonary nodules, and the study mainly involves two technical modules: image segmentation and intelligent detection. The image segmentation is mainly intended to intelligently segment the key parts in the lump such as tracheas, lobes and blood vessels and the like, and establish the model and exhibits the model in a three-dimensional image, so as to help the clinical doctors and imaging doctors to better analyze the lump structure and make preparations for the surgery. The image segmentation has already very matured technique and algorithm. However, the image segmentation has been mainly applied to very traditional algorithm, for example, the cascade model algorithm, and thus fails to bring the intelligent analyzer into full play. In addition, the analysis system with respect to the image segmentation is merely directed to a very small portion in medical data processing, and has very limited value to the doctors.

The deep learning is a well recognized revolutionary technology in the field of artificial intelligence, has overthrown the traditional application methods in such fields as image recognition, voice recognition and the like, and successfully creates many other breaking-through technical applications: Google image content analysis, Google unmanned vehicle, Google Book, Google Brain and the like.

However, nowadays, in the field of medical data analysis, most of the methods still use the very traditional categorization methods, such as support vector machine and the like, which do not represent the most advanced technology in the field of artificial intelligence at present. For example, in the Patent Application No. CN201110376737.X claiming the similar function, the gradient boosting method is used, which is a method that is most extensively applied in the past decade from 1995 to 2005. However, the gradient boosting method still fails to be the most advanced method in the field of artificial intelligence at present.

In the two-dimensional and three-dimensional image recognition algorithms, the most advanced currently is well known as the deep neural network (DNN) algorithm (the details may be referenced to Bengio-2009, citation: Yoshua Bengio, "Learning Deep Architectures for AI", Foundations and trends® in Machine Learning 2(1), 1-127). In some fields with masses of training data, such as, handwritten digit recognition and traffic light recognition, an accuracy surpassing the accuracy reached by man recognition may be achieved.

In the present invention, the most advanced deep learning algorithm is applied to the medical data analysis, and the deep learning algorithm cooperates with the masses of data to perform modeling and construct the medical data analysis system. In this way, the work load of the doctors is greatly reduced, and the working efficiency of the doctors is improved.

The deep learning mainly includes a model pre-training module and a model fine-tuning module.

The model pre-training module is mainly intended to find a digit expression manner that best represents the medical analysis according to the medical training data. The model application module is a major application module in the intelligent analyzer system, and inputs the to-be-analyzed medical data into the model pre-training module such that the model pre-training module automatically outputs a medical pathological analysis matching the to-be-analyzed medical data.

The present invention is hereinafter described in detail.

The method according to the present invention the following steps:

I. Masses of docketed medical material data of the same category and medical diagnosis data matching the medical material data are acquired as medical training data, and the medical training data is stored in a computer.

The medical training is intended to allow the computer to automatically deduce corresponding medical diagnosis and analysis data from the medical material data.

The medical material data includes relevant information recorded in terms of diagnosis, examination and treatment for patients by doctors at clinical and medical technique stages; and the diagnosis data includes relevant information recorded in terms of preliminary diagnosis, hospital discharge result, disease treatment effect for the patients by the doctors at the clinical and medical technique stages, and textual visit data and tracking visit data documented by the doctors.

For example, in the clinical aspect (surgery and internal medicine):

The medical material data includes: patient information input by the doctor, for example, current disease history, previous disease history, health examination, laboratory and instrument examination, treatment course after being admitted and the like records.

The medical diagnosis data (which is also referred to as the target data) includes: preliminary diagnosis made by the doctor after the patient is admitted, hospital discharge result, disease treatment effect and the like records.

Clinical example:

A patient is admitted; relevant information of the patient is input, for example, age, gender, weight, current disease history, previous disease history, health examination and the like information; the data is integrated and analyzed; and a disease category analysis, admittance suggestion and planed treatment solution are provided for the patient. For example, relevant information of a patient is input, wherein the information includes: 65 years old, male, coughing, chest tightness, recent prevalence, long smoking history, no examination made previously and the like.

In the medical technique aspect (pathological department, examination department, radiological department, nuclear medicine department and the like):

The medical material data includes: raw image data, pathological category, disease-related examination data, specific lesions position, metastasis or multiple metastasis and the like.

The medical diagnosis data includes: visit data and tracking visit data that are drafted by the doctor.

Medical technique example:

Radiological department: raw image data obtained from different body parts by using different imaging examination means is analyzed, such that the intelligent analyzer is capable of recognizing and analyzing lesions, and giving further diagnosis and treatment suggestions. For example, with respect to CT intelligent diagnosis of solitary pulmonary nodule, the intelligent analyzer is capable of retrieving all the raw images with an extremely short period of time, and determining whether the position, size, internal density, and edge morphology of the lesions, and other positions in the image are normal.

II. Image data that is not less than two dimensions in the medical training data with variation values variable is associated with time and space in textual data and corresponding data. In other words, the medical material data and the medical diagnosis data regarding the same case are associated.

III. In the acquired masses of docketed medical material data, the medical training data corresponding to each individual and the variation value are aggregated as unit data.

That is, the medical training data relevant to a person or a series of diseases and the variation values are aggregated as a piece of unit data.

IV. The medical training data is integrated or formatted into a computer-understandable structured data matrix by means of segmentation, association or textual analyzing, and a data feature is extracted from each unit data.

The data feature includes variation values variable of the medical training data in terms of time and space, and various mathematic statistical values of the medical training data.

The data feature includes: variations of the medical training data with the time, for example, a tendency of data rise or falloff; and special variations, a position relationship between one pixel and the next pixel of the image data. The data feature further includes: various mathematic statistical values of the medical training data, for example, comparison values of individual data with other individual data. The data features may be formatted as a computer-understandable structure in the form of a vector, a matrix or an array. Acquisition of the data feature also includes image processing or initial data statistical collection. In image processing, segmenting image content relevant to the medical diagnosis and treatment data is a first step to find the image data feature. In document file processing, term frequency-inverse document frequency (TF-IDF) is a manner for quantifying material retrieval and text mining, which may also be widely applied. The above initial image text processing may greatly facilitate acquisition of the data feature for the computer.

V. The medical training data that is formed to the structured data matrix is imported into a memory module in a computer corresponding to a deep learning model.

VI. An optimization operation is performed for the deep learning model via the computer as follows:

1. setting a basic deep learning framework, and establishing a data model including an input layer, at least one hidden layer and an output layer for the medical training data according to the data feature, wherein the input layer includes several nodes having a data feature, the output layer includes several nodes having a medical diagnosis data feature, and each hidden layer includes several nodes having a mapping relationship with an upper-layer output value;

b. establishing a data model for each node by using a mathematic equation, and presetting a relevant parameter value Ai in the mathematic equation in a manual or random way, wherein an input value of each node in the input layer is the data feature, an input value of each node in the hidden layers and the output layer is an upper-layer output value, and an output value of each node in all the layers is a value of the node experiencing the mathematic equation operation; and

c. initializing the parameter value Ai, comparing the output value of each node in the output layer with the medical diagnosis data feature of the corresponding node, repeatedly rectifying the reference value Ai of each node, and performing the operation cyclically, such that the parameter value Ai of each node that causes a similarity between a similarity between the output value of each node in the output layer and the medical diagnosis data feature to be a locally maximum value is acquired.

The parameter value Ai is optimized by using a non-supervised leaning approach or a supervised leaning approach.

The non-supervised leaning approach employs a denoising autoencoder or a restricted Boltzmann machine for self-learning.

The mathematical equation is a parameter mathematical equation or a non-parameter mathematical equation; wherein the parameter mathematical equation is a linear model, a neural network model or a convolution operation, the non-parameter mathematical equation is an extremum operation equation, and a mathematical model is defined as follows:

$$y=g(X)=fn \circ fn\text{-}1 \circ fn\text{-}2 \circ \ldots \circ f1(X)$$

wherein y denotes a medical diagnosis data feature in the output layer having a dimension of $M_n$, X denotes training material data having a dimension of $M_0$, and $f_1$ to fn denote defined operation equations in each layer, wherein equation $f_i$ of each layer has a dimension of $M_i\text{-}1\text{->}M_i$, equation $f_1$ of the first layer converts the X having the dimension of $M_0$ into an output $Z_1$ having a dimension of $M_1$, $Z_1$ is an input of equation $f_2$ of the second layer, and the like, wherein model $f_i$ of each layer model has a matched parameter group $A_i$.

For example, the logic equation is expressed as follows:

$$y = \frac{\exp(\sum_{m \in M} x_m a_m)}{1 + \exp(\sum_{m \in M} x_m a_m)}$$

For example, the linear equation is expressed as follows:

$$y = \sum_{m \in M} x_m a_m$$

wherein $x_m$ denotes an input value of the equation, y denotes an output of the equation, $a_m$ denotes a basic parameter of the equation.

The parameters $A_1$ to $A_n$ of the deep learning model may be initialized, and the model parameters and the model depth and the like may be randomly set. Alternatively, the initialized parameter model may be selected in any manner.

The operation method is interpreted as follows:

The core of the operation is deep learning based on the supervised learning algorithm, which has been a revolutionary technology in the field of artificial intelligence and machine learning in the past five to ten years. According to the present invention, spatial time sequence variation in the case of lesions scanning is innovatively added on the basis of the DNN algorithm, the image rule of the lesions in the three-dimensional space is fully considered and the recognition probability is improved. In addition, with the constructed model, manual judgment or determination of the doctor may be introduced, such that the processional determination of the doctor incorporates the pure intelligent determination to perform modeling and calculate the lesions probability.

The present invention pertains to the technical field of artificial intelligence. The data operation is finally intended to enable the "training" model to automatically recognize the lesions in the medical imaging, give the probability and mark the lesion, and facilitate the diagnosis of the doctor. Therefore, in the model construction course, masses of data is equivalent to the teaching material, whereas the model framework is a specific flowchart summarized after the algorithm abstracting the specific information. Therefore, in the course of intelligent operation, the masses of data and the intelligent algorithm are indispensable.

The following aspects are introduced hereinafter:

1. Medical training data

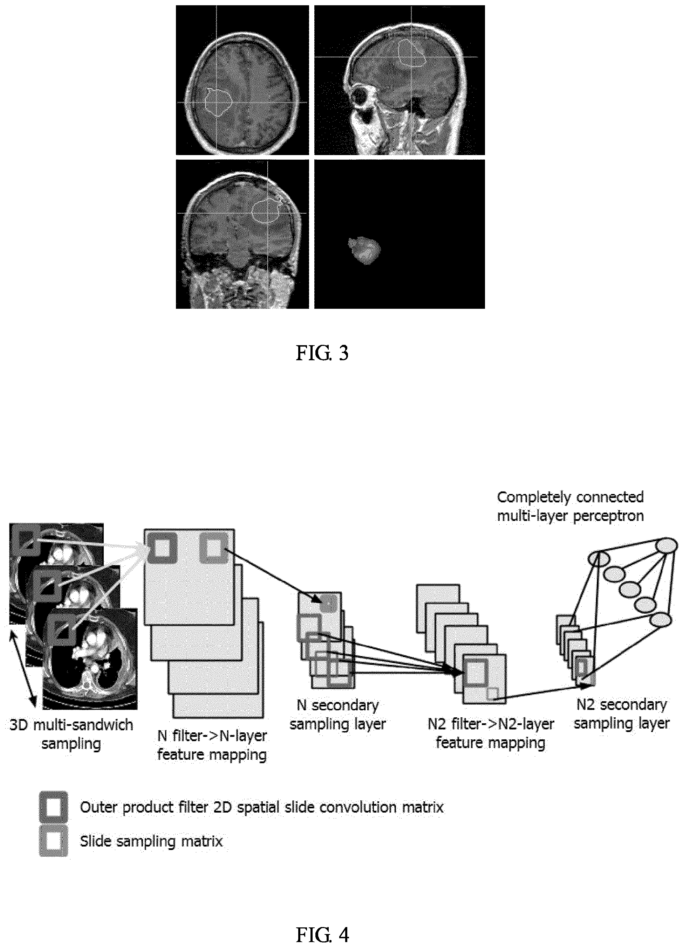

a) Objectively-existent natural data: natural diagnosis and treatment data formed by practice in hospitals. Training data that is closer to the medical diagnosis data generated in the practical application scenarios represents a better training effect. In addition, corresponding requirements are also imposed to the data as the teaching material. These two data sources (that is, the medical material data and the medical diagnosis data) are both indispensable for practice of the present invention. Firstly, raw medical data is needed. For example, many data formats are generally formed upon imaging from medical instruments, such as, .nii.gz, .dcm and the like formats, and all these formats may be represented by multi-dimensional matrix data information before being input to the training model. For example, a magnetic resonance imaging (MRI) image may be a three-dimensional matrix (gray-scale), that is, a two-dimensional gray-scale index and a one-dimensional cross section; or a four-dimensional matrix (RGB), that is, a two-dimensional color index and three color indexes, plus a one-dimensional cross section. Any medical data may be abstracted and simplified as such matrices. Such matrices form a raw data source that is read by the model. As illustrated in FIG. 2 and FIG. 3, image data generated by brain MRI is illustrated. Assume that the system generates a 512 x 512 image from an MRI slice, and 200 slices are generated in one bran scanning, then the data source from one gray-scale brain scanning may be summarized into a data row having a dimension of 512 x 512 x 200=52,428,800. With respect to the model, the number 52,428,800 includes all understood and summarized information of all the bran scanning.

With respect to the non-supervised learning, this type of raw medical matrix is the basic data for modeling. With respect to the supervised learning, an analysis target matching the graph is also needed. The simplest binary analysis information includes a measured lesions body (more complicated information may be the probability of the lesions). Afterwards, more complicated medical data, for example, lesions category, treatment effect, specific position of the lesions and the like information may be integrated. With respect to an even more complicated lesions development prediction system, longitudinal time sequence data of the previous health examinations of the patients may be provided such that the algorithm predicts the development regulations of the medical phenomenon.

b) Simulated data formed via simulation: The simulated data is formed by automatic processing or simulation by a computer, and the simulated data is used as the training data for modeling.

A most typical example of this type of model is the Xbox Kinect system from Microsoft. At the development stage, the basic data of a hand gesture recognition model is all acquired by means of 3D modeling.

In the present invention, the simulated data may be understood as new data that is constructed via deformation, distortion and noise overlapping based on the original medical data. The simulated data is used for the following two reasons. First, addition of the deformed data is favorable to teaching the algorithm to more stably recognize the core variation regulation in the medical data. Second, a general DNN model needs to derive millions of parameters, and in the case of a limited data volume, over-fitting may be inevitably caused, that is, the model over-learns the history data and fails to better summarize and abstract the core variation regulation. Adding the simulated deformed data is equivalent to adding noise in the training process, which may cause the algorithm to better differentiate the noise and the valid information and is favorable to solving the problem of over-fitting.

2. Machine learning algorithm model

The machine learning algorithm is a basic mathematical framework that is used to generalize, and summarize and abstract the information, and this algorithm is mainly intended to express the pattern recognition in a computer-understandable mathematical structure. The training process is estimating the parameters in the model, and upon completion of parameter estimation, the model may become a core part of in the method of the present invention. The machine learning algorithms may be put into two categories, supervised learning and non-supervised learning, according to different objectives. The present invention covers these two categories.

a) Supervised learning: emphasizes the target regulation to be found by manually establishing a model. As described above, in addition to the raw graphic matrix data, the supervised learning algorithm further needs matched analysis result data (for example, the medical diagnosis data).

The present invention mainly involves the following supervised learning algorithms:

i. Deep neural network algorithm

The basic principle of this algorithm is to simulate a brain recognition process. The input of the DNN algorithm is the raw medical data and history analysis results from the doctors, and finally the analysis is automatically completed. The DNN is abstracted and summarized as follows:

$$f(x)=y$$

In the above formula, x denotes raw medical matrix data, and y denotes an intelligent system analysis result. DNN is a mathematical mapping expression formula of the equation $f(x)=y$.

Different assumptions are formulated for f in different supervised learning models. The DNN algorithm simulates the neural structure of the human brain for modeling. A DNN basic mathematical structure based on the graphic data is as illustrated in FIG. 4.

[Note: This is an option of the above described DNN model, wherein the first layer is a convolution layer and the second layer is a maximum pool layer and the like]. The DNN structure may be divided into a plurality of layers from the left graphic raw data to the right analysis result, wherein each layer completes different mathematical operation. The model has totally a plurality of layers of neural network structures. Each layer completes a plurality of parallel inner production operations for the medical data. In the first layer, the most common algorithm is convolution, and the convolution algorithm slide a new equation on a series of input values to output an inner product of the new equation and the original series of data. In the 3D medical graphs, a plurality of convolution blocks are constructed using the algorithm, wherein each convolution block is a 3D matrix. x and y axes of the convolution block cover an equation indicating special variations of an image, whereas z axis of the convolution block covers an equation indicating special variations of the image. Each convolution block matrix slides with the dimension of the data, and calculates an inner product of values in various dimensions of the 3D image and the convolution block. The value obtained by the inner product operation may be understood as a similarity between the data dimension and the convolution block, and the inner product value output by each portion of the data would be an input value of a next layer of neural network. Visually, the parallel convolution matrices are equivalent to specific shapes, and outer and inner calculations of the convolution matrix are equivalent to determining whether different regions are approximate to the specific shape in the data.

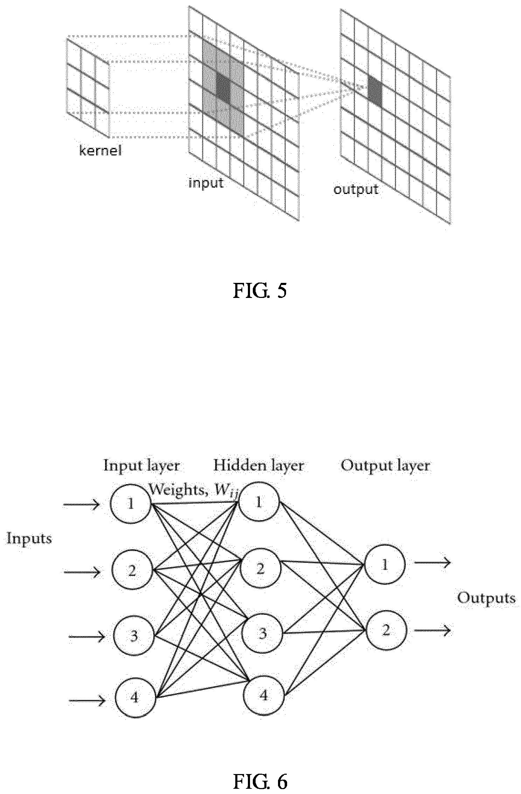

As illustrated in FIG. 5, the second common layer in the DNN model is a layer in which pooling operation (Max Pooling, which is referred to as an MP operation) is performed. In the MP operation, dimension information is synthesized into larger blocks, wherein a Max operation (which calculates a maximum value) is performed in each block. The MP operation mainly simulates the neural network active economic feature in the visual neural network. Within an information framework of a specific range, the most active information unit is allowed to enter the next layer. From the perspective of graphs, the MP operation ensures that the operation result does not change with rotation of the data. From the perspective of operations, the MP operation is equivalent to dimension reduction processing. In combination with the neural network operation in the first layer, the MP operation removes region information having a low similarity to the first layer of convolution block, and reduces the amount of invalid information in each region.

The DNN structure is generally formed by means of combining a convolution layer and a pooling layer, and such operation is repeated to extract the data feature related to the medical diagnosis. Visually, for example, as illustrated in FIG. 4, the intermediate layer algorithm uses a non-linear element formed by the first layer and the second layer, and so on. As such, a more abstract framework element is constructed. With the method according to the present invention, numerous layers of neural networks may be constructed.

Theoretically, on the premise that a large amount of data may be acquired for training, the more repetitions of the combination the convolution layer and the pooling layer, the better. Although the running mechanism of the human brain has not be fully understood, it is well known that the human brain pertains to a very deep and profound neural network structure. Therefore, a deeper and more profound neural network model indicates a stronger power. However, a deeper and more profound neural network structure always has more parameters and is thus hard to be trained and the parameters are also hard to be estimated. As a result, problems of derivative death and over-fitting may be easily caused.

After multiple convolution operations and MP operations are performed in the DNN, the remaining information finally enters an absolutely interconnected multi-layer perceptron (MLP). The basic structure of the perceptron includes two layers of logic regression operations, which is equivalent to assigning values to contributions of the final results of different abstract graphic elements. A final output value of the MLP operation is a medical analysis result of the model. As illustrated in FIG. 6, the perceptron generally has one layer of hidden perception layer, and the variable of each unit is closely interconnected with all the variable in the upper layer, a logic operation is performed in each layer so as to derive the value of the next layer.

b) Non-supervised learning: The concept of the neural network has been available for many years. However, due to the limited data volume and operation capability of the processor, the problem of derivative death is severe, and thus this concept may not be used to solve the practical problem. In a complete model training process, the difference between the output prediction value of the model and the practical value serves as a basis for optimization of the model parameters, and the excessively deep neural network structure fails to push the parameter optimization information to the bottom-layer network, that is, the surface information may not be transferred through layers to the deep neural network structure, which brings difficulties to model training. Especially, in the field of medical data analysis, the medical data is generally large in volume, a complete optimization and search is unrealistic, and the operation difficulty is significantly higher than those in other fields. According to the present invention, the parameters in the model are preliminarily optimized via non-supervised learning, such that the initial conditions of the parameters in the model optimization process become very favorable. In this way, a local minimal value may be quickly found in the model optimization process.

Two most effective non-supervised learning approaches are denoising autoencoder (dAE) and restricted Boltzmann machine (PBM).

i. Denoising autoencoder (hereinafter referred to as dAE)

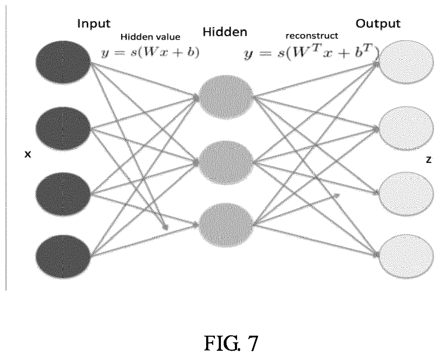

The principle of the autoencoder is finding valid hidden variables of a data variable. As illustrated in FIG. 7, the working principle of the autoencoder is fully illustrated. x is input according to the raw medical data, the autoencoder searches for a hidden element representative y and a parameter W to reflect new data z, and the final intention of the autoencoder is to find the parameter W to minimize the difference between z and x, that is, the autoencoder is finally intended to find parameters that best and completely represent data variable information within limited information. These parameters may be considered as covering a maximum volume of raw data information within the range of the model. With respect to the denoising autoencoder, a large amount of noise is introduced based on the working principle of a simple encoder. Visually, a large amount of noise causes the model to search for more valued potential regulations, without any impact caused by the invalid regulations in the noise. The finally trained parameters become preliminary parameter initial values of the supervised learning, which is equivalent to finding a good starting point for the model at the first step. In this way, parameter optimization for the trained model is greatly accelerated.

iii. Restricted Boltzmann machine (hereinafter referred to as RBM)

The restricted Boltzmann machine achieves an effect that is very similar to the dAE, that is, making preparation for the parameter optimization step in the non-supervised learning. Similar to the dAE, the RBM is also intended to search for a factor orientation that best reflects the raw data variable, and use the factor as an initial parameter of the subsequent supervised learning. However, different from the dAE, the RBM uses an energy probability generation model, not a determined mathematical expression as used in the dAE. From the perspective of operation solving, the dAE is more complicated. However, the probability generation model thereof is essentially more applicable to some modeling scenarios that emphasize event probabilities. With respect to the modeling process, to further reduce the over-fittings, the two non-supervised learning approaches, the RBM and the dAE, would be both used in an integrated learning process.

A further improvement to data optimization is to establish a loss equation or a target equation, and parameter optimization is performed for the supervised learning data model according to the loss equation. The loss equation may be defined as a difference between an analysis result output by the deep learning model in the training data and a practical target variable. During the operation, parameter values in the model are adjusted by means of optimization according to the variations of the loss equation. For example, in an application case of measuring cardiovascular cross-sectional areas in medical imaging, the loss equation may be defined as a difference between a model generated measurement value and a practical value (for example, a variance difference). The parameter values are shifted in each cycle by means of gradient descent parameter optimization, and the parameter optimization is stopped if the parameter optimization cycle satisfies a specific condition (for example, it is defined that the value of the loss equation in the cycle and before the cycle is less than a specific threshold, or the number of cycles exceeds a value), and an optimal value is remained.

The optimization process of the target equation may be expressed as follows:

$$O^* = \min_A O(A, X) = \min_A \{L[Y, g(X, A)] + \lambda R(A)\}$$

In the above formula, $g(x, A)$ denotes an analysis output of basic deep learning, Y denotes a practical value of an analysis target, and $L[Y, g(X, A)]$ is mainly used to calculate the cost caused by the difference between the analysis output of the basic deep learning and the practical value.

$L[Y, g(X, A)] = \sum_i |y_i - g_i(x_i, A)|$ or $L[Y, g(X, A)] = \sum_i [y_i - g_i(x_i, A)]^2$ is commonly used.

R(A) is a regular expression, and is mainly to used to prevent over-fittings of the model.

The most common regular expressions include L1 regular expression $R(A) = \sum_{a \in A} |a|$ and L2 regular expression $R(A) = \sum_{a \in A} a^2$

However, a user may use any other regular expression.

The parameter optimization method may be randomly selected. The most common one is gradient descent. The mathematical expression of steps thereof is as follows:

$$A_{j+1} = A_j - \gamma \nabla O(A_j, X)$$

The parameter is shifted reversely against the differential direction of the target equation at the jth run. Upon repeated multiple runs of shifting, the shifting is stopped if a specific stop condition is satisfied.

A still further improvement to data optimization is data denoising. Noise may be increased to the model or data, to stabilize the model and avoid over-fittings of the data model. For example, in a medical imaging intelligent analyzer, the raw data may be deformed or distorted to cause the model to recognize other valid information except the noise.

A still further improvement to parameter optimization is test sample segmentation. The training data may be further segmented into test samples, and a model is established using the remaining training data. Effectiveness of the model is tested by using the test samples, and the core framework of the deep learning model may be automatically or manually adjusted according to the test result.

VII. To-be-analyzed medical data formed as the structured matrix data is imported into the deep learning model for a medical pathological analysis matching the to-be-analyzed medical data.

VIII. A medical pathological analysis result matching the to-be-analyzed medical data by the deep learning model is output via an output apparatus.

IX. The structured data relevant to the to-be-analyzed medical data and the matched medical pathological analysis result is fed back to the deep learning model to form new training data, such that the deep learning model is further optimized.

An intelligent analyzer for analyzing medical data based on deep learning according to the present invention includes: an input apparatus configured to import medical training data and to-be-analyzed medical data into a computer, a memory module configured to respectively or integrally store the medical training data and the to-be-analyzed medical data, a deep learning model module configured to call the medical training data in the memory module for self-learning, an output apparatus configured to export a medical pathological analysis result matching the to-be-analyzed medical data, and a processor including a CPU and/or a GPU; wherein

the medical training data includes medical material data and medical pathological data matching the medical training data; the medical training data and the to-be-analyzed medical data are a computer-understandable structured data matrix; the self-learning employs a parameter mathematical equation including a linear model, a neural network model, a convolution operation and/or a maximum value operation;

The input apparatus includes a computer device deployed in a hospital or a medical organization, and various medical examination devices and pathological analysis devices that are connected to the computer device, for example, a computer, a color Doppler ultrasound instrument, an X-ray instrument, a synchronous electrocardiograph, a biochemistry analyzer, an immunoassay instrument, a fiber endoscope, an MRI instrument, a CT Doppler diagnostic instrument, a blood pressure meter, a weight scale or the like.

the output apparatus includes a fixed computer output terminal and a mobile intelligent terminal that are deployed in the hospital or the medical organization and connected to the input apparatus, for example, a computer, a medical instrument, a mobile phone or the like.

For resource sharing in a hospital or a medical organization, the intelligent analyzer according to the present invention further is further provided with a network connection module that is connectable to the Internet and Ethernet, wherein the network connection module supports optical fiber connection, WiFi connection or GPRS module connection.

The intelligent analyzer according to the present invention applies the deep learning model obtained by means of training to practice, and forms a complete integrated system. After new medical data (that is, the to-be-analyzed medical data) is generated, the new medical data is combined with the model parameters, and thus an analysis prediction value is obtained. In practical application, the intelligent analyzer serves as an addition plug-in in the analysis process. In different application scenarios, the intelligent analyzer may become an additional plug-in in the medical instrument, or may become an inserted webpage in a picture archiving and communications system (PACS) or a hospital information system (HIS), or may import the generated analysis report to other systems via an Internet interface.

## Claims

1. A method for analyzing medical data based on deep learning, comprising the following steps:

   1) acquiring masses of docketed medical material data of the same category and medical diagnosis data matching the medical material data as medical training data, and storing the medical training data in a computer;
   2) associating image data that is not less than two dimensions in the medical training data with variation values variable with time and space in textual data and corresponding data;
   3) in the acquired masses of docketed medical material data, aggregating the medical training data corresponding to each individual and the variation value as unit data;
   4) integrating or formatting the medical training data into a computer-understandable structured data matrix by means of segmentation, association or textual data mining, and extracting a data feature from each unit data;
   5) importing the medical training data that is formed to the structured data matrix into a memory module in a computer corresponding to a deep learning model;
   6) performing an optimization operation for the deep learning model via the computer as follows:

      a. setting a basic deep learning framework, and establishing a data model comprising an input layer, at least one hidden layer and an output layer for the medical training data according to the data feature, wherein the input layer comprises several nodes having a data feature, the output layer comprises several nodes having a medical diagnosis data feature, and each hidden layer comprises several nodes having a mapping relationship with an upper-layer output value;
      b. establishing a data model for each node by using a mathematic equation, and presetting a relevant parameter value Ai in the mathematic equation in a manual or random way, wherein an input value of each node in the input layer is the data feature, an input value of each node in the hidden layers and the output layer is an upper-layer output value, and an output value of each node in all the layers is a value of the node experiencing the mathematic equation operation; and
      c. initializing the parameter value Ai, comparing the output value of each node in the output layer with the medical diagnosis data feature of the corresponding node, repeatedly rectifying the reference value Ai of each node, and performing the operation cyclically, such that the parameter value Ai of each node that causes a similarity between a similarity between the output value of each node in the output layer and the medical diagnosis data feature to be a locally maximum value is acquired;

   7) importing to-be-analyzed medical data formed as the structured matrix data into the deep learning model for a medical pathological analysis matching the to-be-analyzed medical data; and

8) outputting a medical pathological analysis result matching the to-be-analyzed medical data by the deep learning model via an output apparatus.

2. The method according to claim 1, wherein the parameter value Ai is optimized by using a non-supervised leaning approach.

3. The method according to claim 2, wherein the non-supervised leaning approach employs a denoising autoencoder or a restricted Boltzmann machine for self-learning.

4. The method according to claim 1, wherein the parameter value Ai is optimized by using a supervised leaning approach.

5. The method according to any one of claims 1 to 4, wherein the mathematical equation is a parameter mathematical equation or a non-parameter mathematical equation; wherein the parameter mathematical equation is a linear model, a neuron model or a convolution operation, the non-parameter mathematical equation is an extremum operation equation, and a mathematical model is defined as follows:

$$y = g(X) = fn \circ fn\text{-}1 \circ fn\text{-}2 \circ \ldots \circ f1(X)$$

wherein y denotes a medical diagnosis data feature in the output layer having a dimension of $M_n$, X denotes training material data having a dimension of $M_0$, and $f_1$ to fn denote defined operation equations in each layer, wherein equation $f_i$ of each layer has a dimension of $M_i\text{-}1 \text{->} M_i$, equation $f_1$ of the first layer converts the X having the dimension of $M_0$ into an output $Z_1$ having a dimension of $M_1$, $Z_1$ is an input of equation $f_2$ of the second layer, and the like, wherein model $f_i$ of each layer model has a matched parameter group $A_i$.

6. The method according to claim 1, wherein the medical material data comprises relevant information recorded in terms of diagnosis, examination and treatment for patients by doctors at clinical and medical technical stages; and the diagnosis data comprises relevant information recorded in terms of preliminary diagnosis, hospital discharge result, disease treatment effect for the patients by the doctors at the clinical and medical technical stages, and textual visit data and tracking visit data documented by the doctors.

7. The method according to claim 1, wherein the data features comprises variation values of the medical training data in terms of time and space, and various mathematic statistical values of the medical training data.

8. The method according to claim 1, wherein the structured data relevant to the to-be-analyzed medical data and the matched medical pathological analysis result is fed back to the deep learning model to form new training data.

9. An intelligent analyzer for analyzing medical data based on deep learning, comprising: an input apparatus configured to import medical training data and to-be-analyzed medical data into a computer, a memory module configured to respectively or integrally store the medical training data and the to-be-analyzed medical data, a deep learning model module configured to call the medical training data in the memory module for self-learning, an output apparatus configured to export a medical pathological analysis result matching the to-be-analyzed medical data, and a processor comprising a CPU and/or a GPU; wherein
the medical training data comprises medical material data and medical pathological data matching the medical training data;
the medical training data and the to-be-analyzed medical data are a computer-understandable structured data matrix;
the self-learning employs a parameter mathematical equation comprising a linear model, a neuron model, a convolution operation and/or a maximum value operation; the input apparatus comprises a computer device deployed in a hospital or a medical organization, and various medical examination devices and pathological analysis devices that are connected to the computer device; and
the output apparatus comprises a fixed computer output terminal and a mobile intelligent terminal that are deployed in the hospital or the medical organization and connected to the input apparatus.

10. The intelligent analyzer for analyzing medical data based on deep learning according to claim 9, wherein the intelligent analyzer further comprises a network connection module that is connectable to the Internet and Ethernet, wherein the network connection module supports optical fiber connection, WiFi connection or GPRS module connection.

Analyzer integrated system

FIG. 1

FIG. 2

FIG. 3

Completely connected
multi-layer perceptron

3D multi-sandwich
sampling

N filter->N-layer
feature mapping

N secondary
sampling layer

N2 filter->N2-layer
feature mapping

N2 secondary
sampling layer

Outer product filter 2D spatial slide convolution matrix

Slide sampling matrix

FIG. 4

EP 3 306 500 A1

FIG. 5

FIG. 6

Input   Hidden value   Hidden   reconstruct   Output

$$y = s(Wx + b)$$   $$y = s(W^T x + b^T)$$

x

z

FIG. 7

# INTERNATIONAL SEARCH REPORT

| International application No. |
| --- |
| **PCT/CN2016/084000** |

## A. CLASSIFICATION OF SUBJECT MATTER

G06F 19/00 (2011.01) i

According to International Patent Classification (IPC) or to both national classification and IPC

## B. FIELDS SEARCHED

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNABS, DWPI: medical science, depth, learn, medical, pathology, disease, diagnose, hospital, data, analysis, analyze, model

## C. DOCUMENTS CONSIDERED TO BE RELEVANT

| Category* | Citation of document, with indication, where appropriate, of the relevant passages | Relevant to claim No. |
| --- | --- | --- |
| PX | CN 104866727 A (CHEN, Kuan), 26 August 2015 (26.08.2015), claims 1-10 | 1-10 |
| X | CN 103914841 A (SHENZHEN UNIVERSITY), 09 July 2014 (09.07.2014), abstract, and claims 1-8 | 9-10 |
| A | CN 104523266 A (HEBEI UNIVERSITY), 22 April 2015 (22.04.2015), the whole document | 1-10 |
| A | CN 103544392 A (UNIVERSITY OF ELECTRONIC SCIENCE AND TECHNOLOGY OF CHINA), 29 January 2014 (29.01.2014), the whole document | 1-10 |

☐ Further documents are listed in the continuation of Box C.        ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 24 June 2016 (24.06.2016) | **30 June 2016 (30.06.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **JIA, Yong** Telephone No.: (86-10) **62411850** |
| --- | --- |

Form PCT/ISA/210 (second sheet) (July 2009)

## INTERNATIONAL SEARCH REPORT
### Information on patent family members

International application No.

**PCT/CN2016/084000**

| Patent Documents referred in the Report | Publication Date | Patent Family | Publication Date |
|---|---|---|---|
| CN 104866727 A | 26 August 2015 | None | |
| CN 103914841 A | 09 July 2014 | None | |
| CN 104523266 A | 22 April 2015 | None | |
| CN 103544392 A | 29 January 2014 | None | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201110376737X

**Non-patent literature cited in the description**

- **YOSHUA BENGIO.** Learning Deep Architectures for AI. *Foundations and trends® in Machine Learning,* vol. 2 (1), 1-127